# EUROPÄISCHE PATENTANMELDUNG

(11) **EP 4 223 919 A1**
(43) Veröffentlichungstag der Anmeldung: **09.08.2023**
(21) Anmeldenummer: 22155158.3
(22) Anmeldetag: 04.02.2022
(51) Int. Cl.: D04H 1/26, D04H 1/4258, D04H 1/4266, D04H 1/492, D04H 1/732, D21H 11/12, D21H 11/14, D21H 13/08, D21H 27/00, A47L 13/00

(54) **NICHTGEWEBTES FASERVLIESSTOFFMATERIAL**

(71) Anmelder: Wepa Professional GmbH, 59757 Arnsberg (DE)
(72) Erfinder: VAN HOOF, Hendrikus Wilhelmus Johannes Antonius, 6104 AP Koningsbosch (NL)
(74) Vertreter: Schäperklaus, Jochen

(57) **Zusammenfassung**

Die Erfindung betrifft ein nichtgewebtes Faservliesstoffmaterial, umfassend, bezogen auf das Gesamtgewicht des Faservliesstoffmaterials,
a) 10 Gew.-% bis 95 Gew.-% Cellulose-Frischfasern aus Pflanzen der Pflanzenart Miscanthus x giganteus sowie
b) mindestens 5 Gew.-% biologisch abbaubare Regeneratfasern.

## Beschreibung

### Gebiet der Erfindung

Die vorliegende Erfindung betrifft ein nichtgewebtes Faservliesstoffmaterial.

### Technologischer Hintergrund der Erfindung

Nichtgewebte Faservliesstoffmaterialien (englisch: "Non-Wovens") sind aus dem Stand der Technik in unterschiedlichen Ausführungsformen bekannt. Diese Faservliesstoffmaterialien eignen sich für zahlreiche Anwendungen in der Papier- und Textilindustrie. Sie sind üblicherweise einlagig oder mehrlagig ausgeführt und weisen in ihren einzelnen Lagen eine Struktur aus Einzelfasern auf, die zwar miteinander verbunden sind, aber nicht in einer identifizierbaren Weise, wie zum Beispiel bei einem Gestrick. Faservliesstoffmaterialien können zum Beispiel durch Schmelzblasverfahren, durch Spinnverfahren, Nasslegeverfahren (so genannte "Wet-Laid"-Verfahren) oder durch Trockenlegeverfahren (so genannte "Air-Laid"-Verfahren) hergestellt werden. Das Flächengewicht derartiger Faservliesstoffmaterialien wird in der Regel in Gramm pro Quadratmeter (g/m²) und die Faserfeinheit in Denier oder Tex angegeben.

Aus nichtgewebten Faservliesstoffmaterialien, die als Hauptbestandteil Frischfasern aus Holz-Cellulose aufweisen, können insbesondere Einwegtücher für den Einmalgebrauch hergestellt werden, welche zum Beispiel zu Reinigungs- und Wischzwecken verwendet werden können. Weitere Anwendungsgebiete für Einwegtücher, die aus nichtgewebten Faservliesstoffmaterialien hergestellt werden, sind Einwegtrockentücher, die sich zum Beispiel in Waschräumen finden lassen und mit denen ein Benutzer seine Hände abtrocknen kann. In diesem Fall werden mehrere Lagen eines Faservliesstoffmaterials auf Holz-Cellulosebasis durch ein Gewebe aus synthetischen Fasern, insbesondere aus einem thermoplastischen Kunststoff, verstärkt, um dadurch die Nassfestigkeit zu erhöhen. Derartige Einwegtrockentücher stellen zum Beispiel eine Alternative zu aufgerollten Baumwoll-Trockentüchern dar, welche von einem Spender auf eine bestimmte Länge abgerollt werden, damit ein Benutzer seine Hände abtrocknen kann. Anschließend wird das Baumwoll-Trockentuch wieder in den Spender eingezogen. Die aufgerollten Baumwoll-Trockentücher werden nach dem Gebrauch gereinigt und können anschließend wiederverwendet werden.

### Darüber hinaus können aus nichtgewebten

Faservliesstoffmaterialien auch vorbefeuchtete Einwegtücher (kurz: Feuchttücher) für den Einmalgebrauch hergestellt werden, die insbesondere dann verwendet werden können, wenn Wasser nicht verfügbar ist oder nicht in bequemer Weise verwendet werden kann. Reisende und Eltern von Kleinkindern finden derartige Feuchttücher häufig besonders praktisch. Eine hohe Nassfestigkeit ist auch bei dieser Anwendung wünschenswert, um zum Beispiel ein Reißen oder Durchstoßen während der Tuchausgabe aus einem Spender beziehungswiese aus einer Verpackung oder während des Gebrauchs wirksam zu verhindern.

Bei der Herstellung werden Faservliesstoffbahnen üblicherweise in einzelne Tücher zugeschnitten. Obwohl die einzelnen Tücher grundsätzlich auch trocken verwendet werden können, werden diese typischerweise mit einer für den vorgesehenen Verwendungszweck geeigneten Chemikalienlotion gesättigt, anschließend gestapelt und in einer fluiddichten Verpackung verpackt, aus der sie zum Gebrauch entnommen werden können. Die Chemikalienlotion beinhaltet oft Bakterizide sowie Emulgatoren, pH-Puffer, Duftstoffe und dergleichen. Die flüssigkeitsdichte Verpackung hält den gesättigten Zustand der Tücher bis zu deren Gebrauch aufrecht.

Ein Nachteil dieser vorstehend beschriebenen Einwegtücher, die aus den oben genannten Faservliesstoffmaterialien hergestellt sind, besteht darin, dass sie nicht biologisch abbaubar und darüber hinaus auch nicht recyclebar sind, da sie stets synthetische, nicht biologisch abbaubare Fasern, die zum Beispiel aus einem thermoplastischen Kunststoff hergestellt sind, enthalten. Somit stellen sie auch für sanitäre Abwassersysteme und Kläranlagen große Probleme dar.

Es ist somit wünschenswert, die vorbefeuchteten oder während des Gebrauchs befeuchteten Tücher, die aus einem Faservliesstoffmaterial hergestellt sind, nach dem Gebrauch über eine sanitäre Einrichtung, ein Abwassersystem oder eine Kläranlage entsorgen zu können. Dabei müssen die vorbefeuchteten oder während des Gebrauchs von einem Benutzer befeuchteten Tücher zwar einerseits eine ausreichende Nassfestigkeit aufweisen, um bei starkem Gebrauch einem Reißen und Durchstoßen zu widerstehen. Andererseits müssen sie aber innerhalb des in einer sanitären Einrichtung, einem Abwassersystem oder einer Kläranlage vorhandenen, sich bewegenden Wassers auch in kleinere Stücke beziehungsweise Fasern zerfallen.

### Zusammenfassende Darstellung der Erfindung

Die vorliegende Erfindung macht es sich zur Aufgabe, ein nichtgewebtes Faservliesstoffmaterial zur Verfügung zu stellen, welches eine verbesserte Spülbarkeit oder eine hohe Festigkeit, insbesondere eine hohe Nassfestigkeit, aufweist und darüber hinaus auch biologisch abbaubar ist.

Die Lösung dieser Aufgabe liefert ein nichtgewebtes Faservliesstoffmaterial mit den Merkmalen des Anspruchs 1. Die Unteransprüche betreffen vorteilhafte Weiterbildungen der Erfindung.

Das erfindungsgemäße nichtgewebte Faservliesstoffmaterial umfasst, bezogen auf das Gesamtgewicht des Faservliesstoffmaterials,
a) 10 Gew.-% bis 95 Gew.-% Cellulose-Frischfasern aus Pflanzen der Pflanzenart Miscanthus x giganteus sowie
b) mindestens 5 Gew.-% biologisch abbaubare Regeneratfasern.

Das erfindungsgemäße Faservliesstoffmaterial ist in vorteilhafter Weise für eine Herstellung in einem Nasslegeverfahren, das in der Fachwelt häufig auch als Wet-Laid-Verfahren bezeichnet wird, oder in einem Trockenlegeverfahren, insbesondere in einem Kardierverfahren, geeignet. Auch eine Kombination aus einem Trockenlegeverfahren mit einem Nasslegeverfahren ist möglich. Vorzugsweise kann das Faservliesstoffmaterial wasserstrahlverfestigt sein. Der Regeneratfaseranteil in der Faserzusammensetzung stellt dem Faservliesstoffmaterial eine hohe Festigkeit, insbesondere eine hohe Nassfestigkeit, zur Verfügung. Die in der erfindungsgemäß vorgesehenen Faserzusammensetzung enthaltenen Cellulose-Frischfasern aus Pflanzen der Pflanzenart Miscanthus x giganteus substituieren dabei Cellulose-Frischfasern aus Holz.

Durch die vorliegende Erfindung wird in einigen Ausführungsformen ein Faservliesstoffmaterial geschaffen, welches eine sehr hohe Trocken- und Nassfestigkeit aufweist, um in einem trockenen oder angefeuchteten Zustand verwendet zu werden, und das darüber hinaus eine ausgezeichnete Handhabbarkeit und ästhetische Gestaltung, ein großes Volumen beziehungsweise eine große Dicke, eine gleichmäßige Flüssigkeitsabgabe, eine geringe Fusselbildung, eine gute Abriebfestigkeit und ausgezeichnete Absorptionseigenschaften erreicht.

Die erhöhte Nassfestigkeit verbessert die Gebrauchstauglichkeit und die Widerstandsfähigkeit gegen das Brechen und Reißen des Faservliesstoffmaterials bei vorbefeuchteten Tüchern oder bei der Befeuchtung trockener Tücher, verbessert die Handhabung der Tücher in automatisierten Spendern und das Entnehmen aus Verpackungen.

Darüber hinaus stellt die vorliegende Erfindung in einigen Ausführungsformen ein nass oder trocken verwendbares Faservliesstoffmaterial bereit, das sich in Wasser bei mäßiger Bewegung, wie sie typischerweise in sanitären Einrichtungen, Abwassersystemen und Kläranlagen vorhanden ist, leicht auflösen kann oder dispergieren oder aufbrechen kann. Die Fähigkeit des Faservliesstoffmaterials, unter mäßiger Bewegung in Wasser aufzubrechen, ist das Ergebnis der Faserzusammensetzung des Faservliesstoffmaterials und der Herstellung durch ein Nasslegeverfahren und eine anschließende Wasserstrahlverfestigung, die ein Aufbrechen in einzelne Fasern oder kleine Materialstücke ermöglichen. Eine erhöhte Nass- oder Trockenfestigkeit ist in dieser Ausführungsform allerdings nicht mehr gegeben.

In einer vorteilhaften Ausführungsform wird vorgeschlagen, dass das Faservliesstoffmaterial darüber hinaus, bezogen auf das Gesamtgewicht des Faservliesstoffmaterials, c) mindestens 5 Gew.-% biologisch abbaubare Fasern anderen Ursprungs umfasst. Unter der Formulierung "biologisch abbaubare Fasern anderen Ursprungs" soll in diesem Zusammenhang verstanden werden, dass es sich hierbei nicht um Cellulose-Frischfasern aus Pflanzen der Pflanzenart Miscanthus x giganteus und auch nicht um biologisch abbaubare Regeneratfasern handelt.

In einer besonders vorteilhaften Ausführungsform kann vorgesehen sein, dass die biologisch abbaubaren Fasern anderen Ursprungs durch Frischfasern hölzerner und/oder nicht-hölzerner Pflanzen oder durch Recyclingfasern oder durch eine Kombination dieser Frischfasern mit den Recyclingfasern gebildet sind.

Vorzugsweise können die Frischfasern durch Cellulosefasern und/oder Holzstofffasern und/oder Hanffasern, insbesondere Manilahanffasern, und/oder Baumwollfasern gebildet sein.

In einer bevorzugten Ausführungsform können die Recyclingfasern durch Fasern aus einem Deinking-Zellstoff gebildet sein. Durch das Einbringen eines Faserstoffanteils aus Recyclingfasern in die Faserstoffzusammensetzung, der durch Fasern aus Deinking-Zellstoff (kurz: DIP) gebildet ist, wird eine noch weiter verbesserte Zersetzung, Dispersion oder Auflösung des nichtgewebten Faservliesstoffmaterials in Wasser erreicht. Der Deinking-Zellstoff (DIP) wird häufig auch als deinkter Zellstoff bezeichnet. Bei einem Deinking-Zellstoff (DIP) handelt es sich um Zellstoff aus Recyclingpapier (Altpapier), das in einem so genannten Deinking-Prozess aufbereitet wird, wodurch Druckfarben und andere unerwünschte Elemente entfernt werden und die Papierfasern freigesetzt werden. Da die in dieser Ausführungsform in das Faservliesstoffmaterial eingebrachten Recyclingfasern eine kürzere Faserlänge als fabrikneue Fasern, die demgegenüber Langfasern bilden, aufweisen, kann eine weiter verbesserte Spülbarkeit (Spülfähigkeit) erreicht werden. Es hat sich in diesem Zusammenhang insbesondere gezeigt, dass sich ein möglichst geringer Anteil biologisch abbaubarer Regeneratfasern und ein höherer Anteil an Recyclingfasern aus Deinking-Zellstoff bei nur leichter Wasserstrahlverfestigung des Faservliesstoffmaterials besonders positiv auf die Spülbarkeit auswirken.

Um die Festigkeit, insbesondere die Nassfestigkeit, des Faservliesstoffmaterials weiter zu erhöhen, wird in einer besonders bevorzugten Ausführungsform vorgeschlagen, dass das Faservliesstoffmaterial, bezogen auf das Gesamtgewicht des Faservliesstoffmaterials, mindestens 10 Gew.-% biologisch abbaubare Regeneratfasern umfasst. Es hat sich in diesem Zusammenhang insbesondere gezeigt, dass sich ein höherer Anteil biologisch abbaubarer Regeneratfasern und eine hohe Wasserstrahlverfestigung zu einer höheren Festigkeit des Faservliesstoffmaterials führen.

In einer vorteilhaften Ausführungsform wird vorgeschlagen, dass die biologisch abbaubaren Regeneratfasern durch Lyocellfasern und/oder Viskosefasern gebildet sind.

In einer besonders vorteilhaften Ausführungsform kann vorgesehen sein, dass das nichtgewebte Faservliesstoffmaterial, bezogen auf das Gesamtgewicht des Faservliesstoffmaterials, 20 Gew.-% bis 70 Gew.-%, vorzugsweise 25 Gew.-% bis 55 Gew.-%, Cellulose-Frischfasern aus Pflanzen der Pflanzenart Miscanthus x giganteus umfasst. Es hat sich gezeigt, dass ein Fasergehalt von 25 Gew.-% bis 55 Gew.-% Cellulose-Frischfasern aus Pflanzen der Pflanzenart Miscanthus x giganteus besonders vorteilhaft ist, um ein Faservliesstoffmaterial zu erhalten, das besonders weich und fest ist und das darüber hinaus auch eine sehr gute Spülbarkeit aufweist.

In einer Ausführungsform wird vorgeschlagen, dass zumindest ein Teil der in der Faserzusammensetzung enthaltenen Cellulose-Frischfasern aus Pflanzen der Pflanzenart Miscanthus x giganteus gebleicht ist.

In einer weiteren Ausführungsform besteht die Möglichkeit, dass zumindest ein Teil der in der Faserzusammensetzung enthaltenen Cellulose-Frischfasern aus Pflanzen der Pflanzenart Miscanthus x giganteus ungebleicht ist. Es hat sich in diesem Zusammenhang überraschend gezeigt, dass sich bei Verwendung vollständig ungebleichter Miscanthus x giganteus-Cellulose (nachfolgend kurz: MxG-Cellulose) in vorteilhafter Weise eine antioxidative und antimikrobielle Wirkung einstellt. Gründe dafür können antioxidante oder antimikrobielle Gruppen des Lignins sein, welches in der MxG-Cellulose enthalten ist. Es hat sich zum Beispiel gezeigt, dass ein Zellstoff aus Miscanthus x giganteus mit einem hohen Feuchtigkeitsgehalt >50 % und einem pH-Wert von 7, der über vier Monate eingelagert wurde, keine visuellen Anzeichen von Degradation oder Schimmelbildung aufwies. Auch der Geruch dieses eingelagerten Zellstoffs war noch normal. Die ausschließliche Verwendung ungebleichter MxG-Cellulose ist daher besonders vorteilhaft.

In einer vorteilhaften Ausführungsform wird vorgeschlagen, dass das Faservliesstoffmaterial ein Flächengewicht von etwa 40 bis etwa 150 Gramm pro Quadratmeter aufweist.

Ein wesentlicher Vorteil des hier vorgestellten Faservliesstoffmaterials besteht in der vollständigen biologischen Abbaubarkeit. Das Faservliesstoffmaterial erreicht eine vollständige biologische Abbaubarkeit durch die ausschließliche Verwendung biologisch abbaubarer Fasern, wie zum Beispiel Cellulosefasern, Holzstoff, MxG-Cellulose, Manilahanf, Baumwolle, Viskose, Lyocell, DIP. Die vorstehende Aufzählung biologisch abbaubarer Fasern ist lediglich beispielhaft und daher nicht als abschließend zu verstehen. Das Faservliesstoffmaterial ist in einigen Ausführungsformen für die Entsorgung und den vollständigen biologischen Abbau in sanitären Einrichtungen, Abwassersystemen und Kläranlagen geeignet.

Das Faservliesstoffmaterial weist im Vergleich zu einem herkömmlichen Faservliesstoffmaterial auf Holz-Cellulosebasis eine verbesserte Weichheit auf. Bestimmte Ausführungsformen, welche DIP enthalten, benötigen weniger Lotion, um vollständig getränkt zu werden, wodurch der Einsatz von Lotion und das Gewicht des Gesamtprodukts verringert werden können.

Das Faservliesstoffmaterial benötigt in vorteilhafter Weise auch keine speziellen Imprägnierlotionen, um die Kohäsion zu erhalten oder den Zerfall in einer Wasserumgebung zu fördern. Das Faservliesstoffmaterial behält seine Nasszugfestigkeit und Dispergierbarkeit in Lösungen mit einem pH-Wert im Bereich von etwa 3 bis etwa 11 bei. Somit kann das Faservliesstoffmaterial mit einer breiten Palette von Lotionschemikalien für ganz unterschiedliche Verwendungszwecke, insbesondere zur Reinigung und Körperpflege, imprägniert werden, was für Hersteller von Tüchern und Endverbraucher sehr vorteilhaft ist.

Gemäß einem weiteren Aspekt betrifft die vorliegende Erfindung ein Tuch, welches aus einem nichtgewebten Faservliesstoffmaterial hergestellt ist. Das erfindungsgemäße Tuch zeichnet sich dadurch aus, dass das Faservliesstoffmaterial nach einem der Ansprüche 1 bis 12 ausgeführt ist. Das erfindungsgemäße Tuch ist für den einmaligen Gebrauch vorgesehen und kann zum Beispiel bei der Verwendung befeuchtet werden.

In einer vorteilhaften Ausführungsform wird vorgeschlagen, dass das Tuch mit einer chemischen Lotion befeuchtet ist. Somit stellt die vorliegende Erfindung auch ein vorbefeuchtetes Tuch, insbesondere ein Wischtuch, zur Verfügung, das aus dem erfindungsgemäßen Faservliesstoffmaterial hergestellt ist und mit einer chemischen Lotion befeuchtet, insbesondere gesättigt, ist; wobei das vorbefeuchtete Tuch zumindest in den meisten pH-Bereichen in der Lage ist, seine Nassfestigkeit zu behalten. Die chemische Lotion beinhaltet zum Beispiel Bakterizide sowie Emulgatoren, pH-Puffer und Duftstoffe.

Das hier vorgestellte Tuch, das aus dem erfindungsgemäßen Faservliesstoffmaterial hergestellt ist, weist eine hohe Trocken- und Nassfestigkeit auf, um in einem trockenen, vorbefeuchteten oder angefeuchteten Zustand verwendet zu werden. In einigen Ausführungsformen ist es dazu in der Lage, sich bei mäßiger Bewegung in bewegtem Wasser nach kurzer Zeit in kleine Stücke und einzelne Fasern aufzulösen, so dass es über sanitäre Einrichtungen, Abwassersysteme und Kläranlagen entsorgt werden kann und dabei vollständig biologisch abgebaut werden kann. Das hier vorgestellte Tuch, das aus dem erfindungsgemäßen Faservliesstoffmaterial hergestellt ist, besteht ausschließlich aus biologisch abbaubaren Fasern und enthält keine synthetischen, nicht biologisch abbaubaren Fasern, insbesondere keine Fasern aus einem thermoplastischen Kunststoff. Das für die Tuchherstellung verwendete Faservliesstoffmaterial ist nicht auf synthetische Fasern aus einem thermoplastischen Kunststoff angewiesen, um eine hohe Trocken- und Nassfestigkeit zu erreichen.

Wenn für die Herstellung des Tuchs ein Faservliesstoffmaterial mit einem DIP-Anteil verwendet wird, reduziert sich aufgrund eines geringeren Wasserrückhaltevermögens (kurz: WRV) der Einsatz von Lotion oder Feuchtigkeit bei der Befeuchtung. Ferner weist das Tuch eine erhöhte Weichheit auf. Weniger Lotion bedeutet weniger Gesamtmasse des Produkts, so dass in vorteilhafter Weise auch CO₂-Emissionen beim Transport reduziert werden können.

### Beschreibung bevorzugter Ausführungsbeispiele

Nachfolgend sollen weitere Merkmale und Vorteile der vorliegenden Erfindung anhand zweier Ausführungsbeispiele näher erläutert werden.

Im Versuchsmaßstab wurden ein feuchtes Toilettenpapier (englisch: "Moist Toilet Tissue", kurz: MTT) und ein feucht oder auch trocken nutzbares Allzweckreinigungstuch (englisch: "Universal Cleaning Wipe", kurz: UCW) aus einem nichtgewebten Faservliesstoffmaterial mit einem Faseranteil aus Cellulose-Frischfasern, die aus Pflanzen der Pflanzenart Miscanthus x giganteus stammen, hergestellt.

Das nichtgewebte Faservliesstoffmaterial wurde im Versuchsmaßstab (Versuchsnummern: V2617/V2623) durch ein Nasslegeverfahren, das häufig auch als "Wet-Laid-Verfahren" bezeichnet wird, erzeugt, danach durch ein so genanntes Spunlacing-Verfahren wasserstrahlverfestigt und im Anschluss daran getrocknet und aufgerollt. Diese Versuchsrollen des nichtgewebten Faservliesstoffmaterials wurden dann in einer Verarbeitungsanlage zu feuchtem Toilettenpapier (MTT, V2617) und zu Allzweckreinigungstüchern (UCW, V2623) verarbeitet.

Weitere Einzelheiten des Herstellungsprozesses sollen nachfolgend näher erläutert werden.

Zunächst wurden in einem Pulper Cellulose-Frischfasern aus Pflanzen der Pflanzenart Miscanthus x giganteus, Zellstofffasern hölzernen Ursprungs und Lyocellfasern zusammen in Wasser aufgelöst und als Suspension in einem Vorratsbehälter gespeichert.

Bei den Versuchen wurden nichtgewebte Faservliesstoffmaterialien mit folgenden Faserzusammensetzungen erzeugt:

V2617: 30% Gew.-% Cellulose-Frischfasern aus Pflanzen der Pflanzenart Miscanthus x giganteus, 50% Gew.-% Fasern aus NBSK ("Northern Bleached Softwood Kraft" / nördlicher Langfasersulfatzellstoff / gebleichter Zellstoff) und 20 Gew.-% Lyocellfasern.

V2623: 30% Gew.-% Cellulose-Frischfasern aus Pflanzen der Pflanzenart Miscanthus x giganteus, 50% Gew.-% Fasern aus ungebleichtem Kraftzellstoff und 20 Gew.-% Lyocellfasern.

Die Gewichtsangaben sind jeweils auf das Gesamtgewicht des Faservliesstoffmaterials bezogen.

Bei den Lyocellfasern handelt es sich um künstlich hergestellte, biologisch abbaubare Regeneratfasern

Diese oben genannten Suspensionen aus allesamt biologisch abbaubaren Fasern und Wasser wurden bis auf eine sehr geringe Konsistenz verdünnt, aus dem Vorratsbehälter zu einem Formieraggregat gepumpt und über einen Stoffauflauf, der aufgrund der Vorvermischung auf eine Kammer eingestellt wurde, auf ein rotierendes Sieb gebracht. Dadurch bildete sich in einem einzelnen Schritt eine gemischte, aber homogene Fasermatte, die im Anschluss durch Hochdruck-Wasserstrahlverfestigung in allen drei Dimensionen wasserstrahlverfestigt wurde (so genanntes "Hydroentanglement").

In beiden Versuchen V2617/V2623 wurden die betreffenden Faservliesstoffmaterialien mit einer spezifischen Energie von 99 Wh/kg beaufschlagt, welche insbesondere die Nassfestigkeiten der Faservliesstoffmaterialien erhöht und darüber hinaus auch dafür sorgt, dass diese sich haptisch nahezu so weich wie ein vergleichbar künstlich aufgebautes Faservliesstoffmaterial anfühlen. Dieser Prozessschritt nennt sich Spunlacing-Prozess und führt schlussendlich zu einem einlagigen Faservliesstoffmaterial, welches in diesem Verfahrensschritt zum Beispiel auch über einen Walzenmantel strukturiert werden kann.

Die auf diese Weise erhaltenen Faservliesstoffmaterialien wurden nachfolgend mittels eines Mehrtrommel-Durchströmtrockners getrocknet und im Anschluss über einen Aufwickler in der entsprechenden Breite als Rollen aufgewickelt. Derartige Rollen können bei Bedarf auch in andere Breiten geschnitten werden.

Das Faservliesstoffmaterial V2617 für das feuchte Toilettenpapier wurde mit einem Flächengewicht von 54,9 g/m² erzeugt und das Faservliesstoffmaterial V2623 für das Universalreinigungstuch wurde mit einem Flächengewicht von 61,7 g/m² erzeugt.

Nachfolgend sollen die Ergebnisse einiger Untersuchungen, die an diesen Faservliesstoffmaterialien durchgeführt wurden, zusammengefasst werden.

Die erlangten Nassfestigkeiten des Faservliesstoffmaterials V2617 beliefen sich auf 9,7 N/50 mm längs (MD) und 7,9 N/50 mm quer (CD). Da die eingesetzte spezifische Energie der Wasserstrahlverfestigung bei beiden Versuchen identisch war, waren nur aufgrund des höheren Flächengewichts bei dem Versuchsmuster V2623 mit 12,3 N/50 mm (MD) und 9,5 N/50 mm (CD) höhere Nassfestigkeiten zu beobachten. Der Bindungsindex für die Nassfestigkeit ((MD+CD)/(2*Grammatur)) lag bei beiden Versuchen bei 0,17 (N/50mm)/(g/m²).

Auch die Trockenfestigkeiten zeigten einen nahezu identischen Bindungsindex mit 0,52 (N/50 mm)/(g/m²). Die entsprechenden Messergebnisse für die Trockenfestigkeiten des Faservliesstoffmaterials V2617 lagen bei 34,6 N/50 mm längs (MD) und 23,5 N/50mm quer (CD) und die des Faservliesstoffmaterials V2623 bei 38,7 N/50mm längs (MD) und 24,8 N/50mm quer (CD).

Ein Vergleich der in der vorstehend beschriebenen Weise hergestellten Faservliesstoffmaterialien mit Faservliesstoffmaterialien, die ähnliche Faserzusammensetzungen nur ohne den Zusatz von Cellulose-Frischfasern aus Pflanzen der Pflanzenart Miscanthus x giganteus (der Anteil Miscanthus x giganteus wurde durch herkömmlichen Zellstoff ersetzt) aufweisen, zeigte, dass der Einsatz von Cellulose-Frischfasern aus Pflanzen der Pflanzenart Miscanthus x giganteus ähnliche Trocken- und Nassfestigkeiten hervorbringt. Es konnten eine Erhöhung der Trockenfestigkeiten mit einem Bindungsindex von 0,44 (N/50 mm)/(g/m²) und für die Nassfestigkeiten ein Bindungsindex von 0,22 (N/50 mm)/(g/m²) festgestellt werden.

Aus den vorstehend beschriebenen Rollen wurden in der weiteren Papierverarbeitung für feuchtes Toilettenpapier und für feuchte Universalreinigungstücher entsprechende Tücher geschnitten, die als Tuchstapel zusammengeführt und von dort aus über Verpackungsmaschinen mit einer entsprechenden chemischen Lotion befeuchtet und in Verkaufseinheiten verpackt wurden. Die fertigen Feuchttücher wurden in einem Qualitätspanel als vergleichbar hochwertig zu synthetischen Faservliesstoffmaterialien eingestuft, aber mit dem Vorteil, ein nachhaltiges, biologisch abbaubares Produkt in der Hand zu halten. Des Weiteren wurden aus dem Fasermaterial des Versuchs V2623 auch trockene Reinigungstücher auf Rollen mit Perforationen hergestellt, die so universell einsetzbar sind.

Alle hergestellten Faservliesstoffvarianten wurden basierend auf der EDANA-Richtlinie (so geannnter "Slosh-Box-Test") bezüglich der Spülbarkeit getestet. Laut dieser Richtlinie müssen Faservliesstoffmaterialien in Wasser zu über 60% auflösbar sein, um diese als spülbar (englisch: "flushable") bezeichnen zu dürfen. Alle getesteten Muster wiesen eine sehr gute Spülbarkeit (> 99%) auf.

Die aus den hier vorgestellten nichtgewebten Faservliesstoffmaterialien hergestellten Produkte sind aufgrund ihrer Zusammensetzung biologisch abbaubar und unterstreichen auch durch die Zusatzeigenschaft der besonders guten Spülbarkeit die Nachhaltigkeit dieser Faservliesstoffmaterialien.

## Patentansprüche

1. Nichtgewebtes Faservliesstoffmaterial, umfassend, bezogen auf das Gesamtgewicht des Faservliesstoffmaterials,
a) 10 Gew.-% bis 95 Gew.-% Cellulose-Frischfasern aus Pflanzen der Pflanzenart Miscanthus x giganteus sowie
b) mindestens 5 Gew.-% biologisch abbaubare Regeneratfasern.

2. Nichtgewebtes Faservliesstoffmaterial nach Anspruch 1,
**dadurch gekennzeichnet, dass** das Faservliesstoffmaterial darüber hinaus, bezogen auf das Gesamtgewicht des Faservliesstoffmaterials,
c) mindestens 5 Gew.-% biologisch abbaubare Fasern anderen Ursprungs umfasst.

3. Nichtgewebtes Faservliesstoffmaterial nach Anspruch 2,
**dadurch gekennzeichnet, dass** die biologisch abbaubaren Fasern anderen Ursprungs durch Frischfasern hölzerner und/oder nicht-hölzerner Pflanzen oder durch Recyclingfasern oder durch eine Kombination dieser Frischfasern mit den Recyclingfasern gebildet sind.

4. Nichtgewebtes Faservliesstoffmaterial nach Anspruch 3,
**dadurch gekennzeichnet, dass** die Frischfasern durch Cellulosefasern und/oder Holzstofffasern und/oder Hanffasern, insbesondere Manilahanffasern, und/oder Baumwollfasern gebildet sind.

5. Nichtgewebtes Faservliesstoffmaterial nach einem der Ansprüche 3 oder 4, **dadurch gekennzeichnet, dass** die Recyclingfasern durch Fasern aus einem Deinking-Zellstoff gebildet sind.

6. Nichtgewebtes Faservliesstoffmaterial nach einem der Ansprüche 1 bis 5, **dadurch gekennzeichnet, dass** das Faservliesstoffmaterial, bezogen auf das Gesamtgewicht des Faservliesstoffmaterials, mindestens 10 Gew.-% biologisch abbaubare Regeneratfasern umfasst.

7. Nichtgewebtes Faservliesstoffmaterial nach einem der Ansprüche 1 bis 6, **dadurch gekennzeichnet, dass** die biologisch abbaubaren Regeneratfasern durch Lyocellfasern und/oder Viskosefasern gebildet sind.

8. Nichtgewebtes Faservliesstoffmaterial nach einem der Ansprüche 1 bis 7, **dadurch gekennzeichnet, dass** das nichtgewebte Faservliesstoffmaterial, bezogen auf das Gesamtgewicht des Faservliesstoffmaterials, 20 Gew.-% bis 70 Gew.-%, vorzugsweise 25 Gew.-% bis 55 Gew.-% Cellulose-Frischfasern aus Pflanzen der Pflanzenart Miscanthus x giganteus umfasst.

9. Nichtgewebtes Faservliesstoffmaterial nach einem der Ansprüche 1 bis 8, **dadurch gekennzeichnet, dass** zumindest ein Teil der in der Faserzusammensetzung enthaltenen Cellulose-Frischfasern aus Pflanzen der Pflanzenart Miscanthus x giganteus gebleicht ist.

10. Nichtgewebtes Faservliesstoffmaterial nach einem der Ansprüche 1 bis 9, **dadurch gekennzeichnet, dass** zumindest ein Teil der in der Faserzusammensetzung enthaltenen Cellulose-Frischfasern aus Pflanzen der Pflanzenart Miscanthus x giganteus ungebleicht ist.

11. Nichtgewebtes Faservliesstoffmaterial nach einem der Ansprüche 1 bis 10, **dadurch gekennzeichnet, dass** das Faservliesstoffmaterial ein Flächengewicht von etwa 40 bis etwa 150 Gramm pro Quadratmeter aufweist.

12. Nichtgewebtes Faservliesstoffmaterial nach einem der Ansprüche 1 bis 11, **dadurch gekennzeichnet, dass** das Faservliesstoffmaterial durch ein Nasslegeverfahren oder durch ein Trockenlegeverfahren oder durch eine Kombination eines Trockenlegeverfahrens mit einem Nasslegeverfahren hergestellt ist.

13. Tuch, welches aus einem nichtgewebten Faservliesstoffmaterial hergestellt ist, **dadurch gekennzeichnet, dass** das nichtgewebte Faservliesstoffmaterial nach einem der Ansprüche 1 bis 12 ausgeführt ist.

14. Tuch nach Anspruch 13, **dadurch gekennzeichnet, dass** das Tuch mit einer chemischen Lotion befeuchtet ist.
